# EUROPÄISCHE PATENTSCHRIFT

(11) **EP 1 402 008 B1**
(45) Veröffentlichungstag und Bekanntmachung des Hinweises auf die Patenterteilung: **24.08.2005**
(21) Anmeldenummer: 02782420.0
(22) Anmeldetag: 01.06.2002
(51) Int. Cl.: C12N 9/00

(54) **VERFAHREN ZUR AUFREINIGUNG EINES ENZYMS UND HIERNACH HERGESTELLTES, AUFGEREINIGTES ENZYM SOWIE VERWENDUNG DES ENZYMS**
METHOD FOR PURIFYING AN ENZYME, A PURIFIED ENZYME PRODUCED THEREBY, AND USE OF THIS ENZYME
PROCEDE DE PURIFICATION D'UNE ENZYME, ENZYME AINSI PRODUITE ET PURIFIEE ET UTILISATION DE CETTE ENZYME

(30) Priorität: 02.07.2001 DE 10131994; 14.07.2001 DE 10134347
(43) Veröffentlichungstag der Anmeldung: 31.03.2004
(73) Patentinhaber: Nordmark Arzneimittel GmbH & Co.KG, 25436 Uetersen (DE)
(72) Erfinder: KURFÜRST, Manfred, 25436 Moorrege (DE); SCHMIDBAUER, Stefan, 35094 Lahntal (DE)
(74) Vertreter: Richter, Joachim
(86) Internationale Anmeldenummer: PCT/EP2002/006021
(87) Internationale Veröffentlichungsnummer: WO 2003/004628

(56) Entgegenhaltungen:
- US-A- 5 332 503
- KLOECK GERD ET AL: "Fractions from commercial collagenase preparations: Use in enzymic isolation of the islets of Langerhans from porcine pancreas." CELL TRANSPLANTATION, Bd. 5, Nr. 5, 1996, Seiten 543-551, XP001146029 ISSN: 0963-6897
- BOND M D ET AL: "PURIFICATION AND SEPARATION OF INDIVIDUAL COLLAGENASES EC-3.4.23.3 OF CLOSTRIDIUM-HISTOLYTICUM USING RED DYE LIGAND CHROMATOGRAPHY" BIOCHEMISTRY, Bd. 23, Nr. 13, 1984, Seiten 3077-3085, XP002232416 ISSN: 0006-2960
- ROUNDS M A ET AL: "Poly(styrene-divinylbenzene)-based strong anion-exchange packing material for high-performance liquid chromatography of proteins." JOURNAL OF CHROMATOGRAPHY. NETHERLANDS 26 JUN 1987, Bd. 397, 26. Juni 1987 (1987-06-26), Seiten 25-38, XP002087598 ISSN: 0021-9673
- LEONARD M ET AL: "Polyvinyl alcohol-coated macroporous polystyrene particles as stationary phases for the chromatography of proteins." JOURNAL OF CHROMATOGRAPHY B BIOMEDICAL APPLICATIONS, Bd. 664, Nr. 1, 1995, Seiten 39-46, XP004043704
- NASH D C ET AL: "Modification of polystyrenic matrices for the purification of proteins Effect of the adsorption of poly(vinyl alcohol) on the characteristics of poly(styrene-divinylbenzene) beads for use in affinity chromatography" JOURNAL OF CHROMATOGRAPHY A, ELSEVIER SCIENCE, NL, Bd. 758, Nr. 1, 10. Januar 1997 (1997-01-10), Seiten 53-64, XP004034014 ISSN: 0021-9673

## Beschreibung

### Anwendungsgebiet

Die Erfindung betrifft ein Verfahren zur Aufreinigung mindestens eines in einem Fermentationsüberstand von *Clostridium histolyticum* enthaltenen Enzyms und ein hiemach hergestelltes, aufgereinigtes Enzym sowie seine Verwendung.

### Stand der Technik

Das Bakterium *Clostridium histolyticum* bildet bei Kultivierung in peptonhaltigem Nährmedium extrazellulär ein komplexes Enzymgemisch, das Kollagenasen, verschiedene proteolytische Enzyme sowie niedermolekulare Bestandteile enthält. Als Hauptbestandteile wurden Typ I und Typ II Kollagenasen (Clostridiopeptidase A, EC 3.4.24.3) mit Molekulargewichten im Bereich von 65 bis 125 kD und isoelektrischen Punkten zwischen 5 und 6,5 beschrieben (Bond, van Wart; Biochemistry, 1984, 23, 3077-3085). Weitere Hauptbestandteile sind die als Heterodimer vorkommende SH-Protease Clostripain (Clostridiopeptidase B, EC 3.4.22.8) mit einem Molekulargewicht von zirka 59 kD und die schlecht charakterisierte so genannte neutrale Protease (Caseinase) mit einem mittels MALDI-TOF bestimmten Molekulargewicht von 34,5 kD.

Kollagenasen sind Enzyme, die Peptidbindungen des Faserproteins Kollagen spalten. Sie finden biochemische und medizinische Anwendung, beispielsweise um Zellen oder Zellverbände von Geweben zu isolieren. Typ I und Typ II Kollagenasen unterscheiden sich in ihrer Aktivität hinsichtlich hochmolekularem Kollagen und kleinen synthetischen Substraten. Während Typ I Kollagenasen bevorzugt hochmolekulares Kollagen spalten, reagieren Typ II Kollagenasen vorwiegend mit synthetischen Substraten, wie zum Beispiel PZ-Pro-Leu-Gly-Pro-D-Arg (Wünsch, Heidrich; Z. Physiol. Chem. 333, 1963, 149-151), His-Pro (Nordwig, Wünsch; Z. Physiol. Chem. 316, 1959, 287) oder Phe-Ala-Leu-Gly-Pro-Ala (van Wart, Steinbrink; Anal. Biochem. 113, 1981, 356-365). Beide Kollagenasetypen lassen sich auch mittels reversed phase Chromatographie (RPC) eindeutig unterscheiden.

Die US 5,332,503 beschreibt ein Verfahren zur chromatographischen Reinigung von Kollagenase aus *Clostridium histolyticum*. Dieses Chromatographieverfahren umfasst unter anderem einen Gelfiltrationsschritt sowie eine Farbstoff-Liganden-Affinitätschromatographie unter Verwendung von Reaktivrot-Agarosegel. Das Verfahren weist entscheidende Nachteile auf, um Kollagenase für pharmazeutische Zwecke unter GMPkonformen Bedingungen herzustellen. So birgt das im Prozess verwendete Reaktivrot-Agarosegel die Gefahr eines so genannten Ausblutens des Chromatographiematerials und einer damit verbundenen toxikologischen Problematik. Des Weiteren sind Gelfiltrationsschritte grundsätzlich zeitaufwendig und kostspielig und bieten wenig effektive cleaningin-place (CIP) Möglichkeiten. Dies erschwert den Einsatz dieses Verfahrens im großtechnischen Maßstab. Femer erfordert das Verfahren die Verwendung von Detergentien, wodurch ein hoher Aufwand zur Reinigungsvalidierung notwendig wird und unerwünschte Veränderungen des Endproduktes hervorgerufen werden können. Schließlich erlaubt das Verfahren keine Trennung der Typ I und Typ II Kollagenasen.

Aus den Dokumenten US 5,830,741 und US 5,952,215 ist ein Aufreinigungsverfahren zur Trennung von Typ I und Typ II Kollagenase bekannt, das eine Farbstoff-Liganden-Affinitätschromatographie, eine Kationenaustauschchromatographie und eine Anionenaustauschchromatographie umfasst. Auch hier ist die Gefahr des Ausblutens des in der Affinitätschromatographie eingesetzten Farbstoffes gegeben. Ferner erlauben die verwendeten Chromatographiematerialien nur verhältnismäßig geringe Flussraten. Zudem erfolgen alle chromatographischen Schritte mittels Gradientenelution, wobei die an den Chromatographiematerialien gebundenen Enzyme durch lineare Salzkonzentrations- und/oder pH-Gradienten eluiert werden. Im Resultat ist das bekannte Verfahren daher äußerst zeitaufwendig.

Durch die US-A-5,332,503 ist ein Verfahren zur Reinigung von Rohkollagenase bekannt. Dieses Kollagenasereinigungsverfahren umfasst die Bereitung einer stabilisierten Rohkollagenaselösung, die Kollagenase, Pigment, Toxine, Bakterienmaterialien und Verunreinigungen durch proteolytische Enzyme einschließlich Clostripain, Trypsin und Caseinase enthält. Die stabilisierte Kollagenaselösung wird dabei auf Hydroxylapatitsäulenmaterial aufgebracht. Pigment und Caseinase werden mit einer ersten Lösung von ungefähr 0,05 M bis ungefähr 0,3 M Phosphatpuffer eluiert und dann Kollagenase, Trypsin und Clostripain mit einer zweiten Lösung von ungefähr 0,35 M bis ungefähr 0,5 M Phosphatpuffer, um eine erste aufgefangene Lösung zu erhalten. Die erste aufgefangene Lösung wird dann auf Gelfiltermaterial aufgebracht und Kollagenase und Closfripain werden mit einer pH-neutralen Pufferlösung eluiert, um eine zweite aufgefangene Lösung zu erhalten. Die zweite aufgefangene Lösung wird dann auf Reactive-Red-120-Agarosesäulenmaterial aufgebraucht und Kollagenase mit einer pH-neutralen Pufferlösung eluiert, um gereinigte Kollagenase zu erhalten. Das Verfahren liefert extrem reine Kollagenase in hoher Ausbeute bei verringertem Verbrauch an Eluierlösungen und vermeidet unkalkulierbare Gradientenelutionstechniken.

Danach weist dieses Verfahren folgende Schritte auf:
- Einbringung einer stabilisierten Kollagenaserohlösung in eine Säule die Hydroxylapatitsäulenmaterial enthält:

- Elution von Pigment und Caseinase aus dem Hydroxylapatitsäulenmaterial mit einer ersten Lösung von ungefähr 0,05 M bis ungefähr 0,3 M Phosphat, gepuffert auf einen pH-Wert von ungefähr 6 bis ungefähr 8, und einem nichtionischen Tensid;
- Elution von Kollagenase, Clostripain und Trypsin mit einer zweiten Lösung von ungefähr 0,35 M bis ungefähr 0,5 M Phosphat, gepuffert auf einen pH-Wert von ungefähr 6 bis ungefähr 8, und einem nichtionischen Tensid, um eine erste aufgefangene Lösung zu erhalten; .
- Einbringen der genannten ersten aufgefangenen Lösung in eine Säule, die ein Gelfiltermaterial enthält;
- Elution von Kollagenase und Clostripain mit einer dritten Lösung, die einen pH-neutralen Puffer enthält, um eine zweite aufgefangene Lösung zu erhalten;
- Einbringen der genannten zweiten aufgefangenen Lösung in eine Säule, die Reactive-Red-120-Agarosematerial enthält; und Elution von Kollagenase mit einer vierten Lösung, die einen pH-neutralen Puffer enthält, um eine Lösung zu erhalten, die gereinigte Kollagenase enthält.

Bei diesem Verfahren muss von einer stabilisierten Rohkollagenase ausgegangen werden.

Die Literaturstelle KLOECK GERD ET AL: "Fractions from commercial collagenase preparations: Use in enzymic isolation of the islets of Langerhans from porcine pancreas." CELL TRANSPLANTATION, Bd. 5, Nr. 5, 1996, Seiten 543-551, XP001146029 ISSN: 0963-6897 befasst sich mit der Transplantation isolierter Langerhans-Inseln zur Behandlung von Diabetes mellitus ist. Die Isolierung von Inseln aus Pankreas erfordert die spezifische Auflösung des Gewebes. Kommerziell erhältliche Kollagenasen aus *Clostridium histolyticum* werden zu diesem Zweck verwendet. Die Wirksamkeit dieser kommerziell erhältlichen Enzyme ist jedoch nicht vorhersehbar und schwankt erheblich zwischen Lieferanten und sogar von Lieferung zu Lieferung. Dies beruht hauptsächlich auf Unterschieden in ihrer spezifischen Kollagenaseaktivität und auf der Anwesenheit anderer lytischer Enzyme, sowie auf anderen Verunreinigungen. Dabei wurde Free-Flow-Zonen-Elektrophorese (FFZE) angewandt, um die wirksamen Proteinbestandteile von unerwünschten Bestandteilen zu trennen und um ein verdauendes Enzymgemisch mit kontrollierter Zusammensetzung lytischer Aktivitäten herzustellen. Eine Fraktionierung von Rohkollagenasen durch FFZE führte dabei zu teilweise gereinigten Proteinfraktionen, in denen Kollagenase- und Trypsin-Aktivität angereichert waren, und die nur Spuren neutraler Protease enthielen. Diese Präparate sollen sich als sehr wirksam bei einem *in vitro*-Versuch zur Freilegung lebensfähiger Inseln aus Schweine-Pankreas herausgestellt haben. Zur Maßstabvergrößerung der Erzeugung dieser Kollagenasen mit definierter Enzymzusammensetzung wurden zwei verschiedene Lieferungen einer kommerziell erhältlichen Kollagenase aus *Clostridium histolyticum* (eine Lieferung, die für die Insel-Isolierung wirksam war, und eine andere unwirksame) unter Anwendung von FPLC-Chromatographie auf Hydroxyapatit fraktioniert. Dabei war eine hohe Wirksamkeit bei der Insel-Freilegung aus Pankreas-Gewebe mit hoher spezifischer Trypsin- und Kollagenaseaktivität und geringem Anteil an neutraler Protease verbunden. Das in dieser Untersuchung entwickelte Chromatographieprotokoll setzte eine unwirksame Kollagenaselieferung in ein Präparat um, das erfolgreiche Insel-Isolierung ermöglichte.

Der Literaturstelle BOND M D ET AL: "PURIFICATION AND SEPARATION OF INDIVIDUAL COLLAGENASES EC-3.4.23.3 OF CLOSTRIDIUM-HISTOLYTICUM USING RED DYE LIGAND CHROMATOGRAPHY" BIOCHEMISTRY, Bd. 23, Nr. 13, 1984, Seiten 3077-3085, XP002232416 ISSN: 0006-2960 ist die Reinigung und Abtrennung einzelner Kollagenasen von *Clostridium histolyticum* unter Anwendung von Affinitätschromatographie mit rotem Farbstoff zu entnehmen. Dabei wird wie folgt vorgegangen:
Sechs im Kulturfiltrat von *Clostridium histolyticum* anwesende Kollagenasen wurden bis zur Homogenität gereinigt. Vermittels Chromatographie über Hydroxylapatit, Sephacryl S-200 und L-Arginin-Affi-Gel 202 wurde das braune Pigment und der größte Teil der kontaminierenden Proteinasen entfernt, die gegen Casein, Benzoyl-L-arginin-ethylester, und Elastin wirksam sind. Reactive-Red-120-Farbstoffaffinitätschromatographie trennt die Kollagenasen, die sehr ähnliche physikalisch-chemische Eigenschaften haben, in vier Fraktionen auf. Die endgültige Reinigung erfolgt durch Chromatograhie über DEAE-Zellulose und SP-Sephadex. Alle sechs Kollagenasen, mit α, β, γ, δ, ε und ζ in der Reihenfolge ihrer Reinigung bezeichnet, zeigen hohe Aktivität gegen Kollagen und weisen keine anderen proteolytischen Aktivitäten auf. Jede stellt ein einzelnes Band auf Natriumdodecylsulfat-Polyacrylamid-Gelen dar. Zwei unterschiedliche Unterarten der α- und γ-Enzyme sind isoliert worden, die dasselbe Molekulargewicht und dieselbe Aktivität haben, aber unterschiedliche isoelektrische Punkte. Es gibt eine etwas weniger deutliche Mikroheterogenität bei den anderen Kollagenasen. Aufgrund ihrer Aktivität gegen natürliches Kollagen und gegen das synthetische Peptid 2-Furanacryloyl-L-leucylglycyl-L-prolyl-L-alanin (FALGPA) werden die sechs Kollagenasen in zwei Klassen aufgeteilt. Die Kollagenasen der Klasse I (α, β und γ) haben eine hohe Kollagenaseaktivität und mäßige FALGPA-Aktivität, während die Kollagenasen der Klasse II (δ, ε und ζ) mäßige Kollagenase- und hohe FALGPA-Aktivität aufweisen. Danach wird dieses Reinigungs- und Abtrennungsverfahren unter Anwendung von Affinitätschromatographie mit rotem Farbstoff durchgeführt.

Das in der Literaturstelle ROUNDS M A ET AL: "Poly(styrenedivinylbenzene)-based strong anion-exchange packing material for highperformance liquid chromatography of proteins." JOURNAL OF CHROMATOGRAPHY. NETHERLANDS 26 JUN 1987, Bd. 397, 26. Juni 1987 (1987-06-26), Seiten 25-38, XP002087598 ISSN: 0021-9673 beschriebene Verfahren befasst sich mit einem Anionenaustauschersäulenmaterial auf Poly(styrol-divinylbenzol)-Basis für die HPLC-Chromatographie von Proteinen, das wie folgt erhalten wird: Eine adsorbierte Polyethylenimin-Beschichtung wurde auf sulphoniertes, makroporöses, kleinteiliges Poyl(styrol-divinylbenzol) aufgetragen. Die chemischen, physikalischen und chromatographischen Eigenschaften des sich ergebenden starken Anionenaustauschersäulenmaterials wurden sorgfältig bestimmt. Die dynamische Bindungskapazität des experimentellen Materials war mit der von Siliziumdioxid großen Porendurchmessers vergleichbar. Gute Wiedergewinnung von Proteinmasse und Enzymaktivität wurden erreicht. Die neue Säule war gegen verschiedene Reinigungsverfahren und langdauemden Kontakt mit wässriger Base widerstandsfähig. Die Rückhaltezeiten in der Säule auf Polystyrolbasis waren denen in einer Siliziumdioxidsäule und einer kommerziell vertriebenen, polymeren starken Anionenaustauschersäule ähnlich. Die chromatographische Auflösung des Säulenmaterials war derjenigen gleich oder höher, die die anderen beiden Säulenmaterialien liefern.

Die Literaturstelle LEONARD M ET AL: "Polyvinyl alcohol-coated macroporous polystyrene particles as stationary phases for the chromatography of proteins." JOURNAL OF CHROMATOGRAPHY B BIOMEDICAL APPLICATIONS, Bd. 664, Nr. 1, 1995, Seiten 39-46, XP004043704 befasst sich mit Polyvinylalkohol beschichteten makroporösen Polystyrolteilchen als stationäre Phasen für die Chromatographie von Proteinen und offenbart ein Verfahren zur Hydrophilisierung makroporöser Poly(styrol-divinylbenzol)(PS-DVB)-Körner durch Adsorption von Polyvinylalkohol (PVA) wird beschrieben). PVA adsorbiert stark an PS-DVB-Oberflächen, desorbiert jedoch teilweise wieder, wenn er Proteinlösungen ausgesetzt wird. Um dieses Problem zu überwinden, wurde die adsorbierte Polymerschicht durch Vemetzung stabilisiert. Wir berichten über die Wirkung von Polymeradsorption und Vemetzungsbedingungen auf die Menge adsorbierten PVA, auf die Stabilität der Polymerschicht und auf die Effektivität der Hydrophilisierung in Hinsicht auf Rinderserumalbumin, ein stark hydrophobes Protein. Die Eigenschaften der beschichteten Träger wurden auch durch Größenausschluss-Chromatographie bestimmt. Es wurde gezeigt, dass die PVA-Beschichtung hydrophobe Interaktionen stark verringert. Es wurde festgestellt, dass sich die Porengröße modifizierter PS-DVB-Teilchen erheblich mit wachsenden Mengen an adsorbiertem PVA verringert.

Die Literaturstelle NASH D C ET AL: "Modification of polystyrenic matrices for the purification of proteins Effect of the adsorption of poly(vinyl alcohol) on the characteristics of poly(styrene-divinylbenzene) beads for use in affinity chromatography" JOURNAL OF CHROMATOGRAPHY A, ELSEVIER SCIENCE, NL, Bd. 758, Nr. 1, 10. Januar 1997 (1997-01-10), Seiten 53-64, XP004034014 ISSN: 0021-9673 offenbart die Modifizierung von Polystyrolmatrizen zur Reinigung von Proteinen und befasst sich mit der Wirkung der Adsorption von Poly(vinylalkohol) auf die Eigenschaften von Poly(styrol-divinylbenzol)-Körnem zur Verwendung in der Affinitätschromatographie, wobei wie folgt vorgegangen wird: Eine Poly(styrol-divinylbenzol)-(PSDVB)-Chromatographiematrix, CG1000-sd (TosoHaas) wurde unter Verwendung von Poly(vinylalkohol) (PVA) verändert, um eine Matrix zu schaffen, die für die Anlagerung funktioneller Gruppen zur selektiven Reinigung von Proteinen geeignet ist. Die Eigenschaften der modifizierten Matrix wurden mit einer BET-Stickstoffadsorptions/desorptionstechnik untersucht und es wurde festgestellt, dass die Adsorption von PVA dazu führt, dass die Mikroporen der Körner gefüllt werden, während die Makroporen der Kömer im wesentlichen unverändert blieben. Es fand keine Proteinadsorption an die modifizierten Matrizen statt. Ein Farbstoffligand (Procion Blue MX-R) wurde kovalent an die PVA-PSDVB-Matrix gebunden und die Lysozym-Kapazitäten der PVA-PSDVB-Matrix wurden bestimmt. Die Matrix ist mit kommerziell erhältlichem Blue Sepharose Fast Flow gut vergleichbar, einer Affinitätsmatrix auf der Grundlage vemetzter Agarose. Die Farbstoff-PVA-PSDVB-Matrix soll stabil gegen Sanierung mit Natriumhydroxid sein.

### Aufgabe, Lösung, Vorteil

Vor diesem Hintergrund ist es Aufgabe der vorliegenden Erfindung, ein Verfahren zur Trennung und Aufreinigung mindestens eines extrazellulären Hauptenzyms von *Clostridium histolyticum* bereitzustellen, das die beschriebenen Nachteile des Standes der Technik überwindet.

Diese Aufgabe wird durch ein Verfahren mit den im Anspruch 1 genannten Merkmalen gelöst.

Das erfindungsgemäße Verfahren besteht somit darin, dass die Enzyme des Fermentationsüberstandes durch ein Chromatographieverfahren unter ausschließlicher Verwendung erster Chromatographiematerialien auf Basis von keramischem, Hydroxylapatit und zweiter Chromatographiematerialien auf Styrol/Divinylbenzolbasis aufgetrennt werden, wobei
a.) das Chromatographieverfahren eine erste chromatographische Stufe an gesintertem Hydroxylapatitmaterial, eine zweite chromatographische Stufe an einem Anionenaustauschermaterial auf Styrol/Divinylbenzolbasis und gegebenenfalls eine dritte chromatographische Stufe an einem Kationenaustauschermaterial auf Styrol/Divinylbenzolbasis umfasst, wobei die Elution an dem gesinterten Hydroxylapatitmaterial bei Flussraten von mindestens 200 cm/h und insbesondere von mindestens 300 cm/h, die Elution an dem Styrol/Divinylbenzolmaterial bei Flussraten von mindestens 500 cm/h, insbesondere von mindestens 1000 cm/h, durchgeführt wird,
b.) die erste chromatographische Stufe in mindestens zwei Elutionsstufen durchgeführt wird, wobei neutrale Protease (Caseinase) von Typ 1 und Typ II Kollagenase und Clostripain abgetrennt wird;
c.) eine im wesentlichen von Caseinase befreite Fraktion aus der ersten chromatographischen Stufe der zweiten chromatographischen Stufe mit mindestens zwei Elutionsstufen unterzogen wird, wobei Typ I Kollagenase und Typ II Kollagenase voneinander getrennt werden,
d.) eine Typ I Kollagenase enthaltene Fraktion aus der zweiten chromatographischen Stufe der dritten chromatographischen Stufe mit mindestens zwei Elutionsstufen unterworfen wird, wobei Typ 1 Kollagenase von Clostripain abgetrennt wird,
e.) eine Typ II Kollagenase enthaltene Fraktion aus der zweiten chromatographischen Stufe der dritten chromatograhischen Stufe mit mindestens zwei Elutionsstufen unterworfen wird, wobei Typ 11 Kollagenase von Clostripain abgetrennt wird,
f.) die chromatographischen Stufen bei Temperaturen zwischen 4° und 25° C durchgeführt werden.

Dadurch, dass die Enzyme des Fermentationsüberstandes von *Clostridium histolyticum* durch ein einstufiges oder bevorzugterweise mehrstufiges Chromatographieverfah-ren unter ausschließlicher Verwendung von Chromatographiematerialien auf Styrol/Divinylbenzolbasis und/oder auf Basis von keramischem Hydroxylapatit aufgetrennt werden, lässt sich eine sehr schnelle und kostengünstige Enzymaufreinigung realisieren, wobei Enzyme mit hoher Reinheit erhalten werden, die aufgrund einer Abwesenheit toxikologisch bedenklicher Substanzen insbesondere für den Einsatz in der Pharmazie und/oder Biochemie geeignet sind. Dieses Verfahren kommt ohne produktberührende Schritte aus, was den Einsatz im Pharmaziebereich besonders vorteilhaft macht. Es kann zur Herstellung von sterilen Kollagenaseenzymen genutzt werden. Dabei wird unter Styrol/Divinylbenzol ein Copolymerisat verstanden, das aus mit Divinylbenzol quervemetztem Polystyrol besteht. Durch Substitution dieser Basismatrix mit unterschiedlichsten funktionellen Gruppen wird eine Bindung von Proteinen an das Material und somit ihre Trennung ermöglicht. Abhängig von der Wahl der funktionellen Gruppe können unterschiedliche Bindungs- und Trennungsmechanismen, beispielsweise Kationen- oder Anionenaustauschmechanismen, genutzt werden. Hingegen handelt es sich bei Hydroxyapatit um ein Kalziumphosphat der Summenformel Ca₁₀(PO₄)₆(OH)₂, das eine Proteinbindung aufgrund ionischer elektrostatischer Wechselwirkungen sowie einer stärkeren ionischen Komplexbindung gestattet. Im Detail ist der Bindungsmechanismus von Proteinen an Hydroxylapatit bislang jedoch noch nicht verstanden.

Die verwendeten Chromatographiematerialien gestatten die Einstellung von hohen Flussraten bei guten Trenneigenschaften, so dass sich sehr kurze Aufreinigungszeiten ergeben. Dies gilt insbesondere, wenn gemäß einer bevorzugten Ausgestaltung der Erfindung gesintertes, also keramisches Hydroxylapatit verwendet wird. Die Verwendung von keramischem Hydroxylapatit besitzt gegenüber nichtgesintertem (kristallinem) Hydroxylapatit den Vorteil, dass das Material reproduzierbar hergestellt werden kann und aufgrund seiner porösen Struktur nur sehr geringe Rückdrucke erzeugt, wodurch die Gefahr von Säulenbeschädigungen minimiert wird und hohe lineare Flussraten ermöglicht werden. Die Elution an gesintertem Hydroxylapatitmaterial wird vorzugsweise bei Flussraten von mindestens 200 cm/h, insbesondere von mindestens 300 cm/h, durchgeführt. Im Falle des polymeren Styrol/Divinylbenzolmaterials werden bevorzugt sogar Flussraten von mindestens 500 cm/h, insbesondere von mindestens 1000 cm/h, eingestellt. Die Prozesszeiten können auf diese Weise gegenüber bekannten Verfahren erheblich verkürzt werden.

Eine weitere Prozesszeitverkürzung sowie eine Vereinfachung des Prozessaufwandes wird erreicht, indem mindestens eine Chromatographiestufe, vorzugsweise alle Chromatographiestufen, in Form einer Stufenelution durchgeführt wird. Gegenüber einer Gradientenelution kann hierdurch femer eine Einsparung des Elutionsmittels und damit teurer Puffersubstanzen erzielt werden. Die Generierung eines Gradienten ist im Prozessmaßstab außerdem problematisch.

Gemäß einer vorteilhaften Ausgestaltung der Erfindung werden die Chromatographiematerialien derart ausgewählt, dass die einzelnen chromatographischen Schritte ausschließlich auf elektrostatischen Wechselwirkungen und/oder auf Ionenbindung und/oder ionischer Komplexbindung beruhen. Besonders vorteilhaft umfasst das Verfahren mindestens eine anionenaustauschchromatische Stufe, und/oder mindestens eine kationenaustauschchromatische Stufe, und/oder mindestens eine hydoxylapatitchromatische Stufe. Besonders gute Ergebnisse wurden in einem dreistufigen Chromatographieverfahren erzielt, wobei eine erste Stufe an gesintertem Hydroxylapatitmaterial, eine zweite Stufe an einem Anionenaustauschermaterial auf Styrol/Divinylbenzolbasis und eine dritte chromatographische Stufe an einem Kationenaustauschermaterial auf Styrol/Divinylbenzolbasis durchgeführt wird, vorzugsweise in der genannten Reihenfolge, jedoch auch andere Reihenfolgen sind möglich; auch die Stufenanzahl kann variieren.

Vorzugsweise umfasst das erfindungsgemäße Verfahren somit keine zeitaufwendigen und kostenintensiven Gelfiltrationsschritte, wodurch die Gefahr eines Selbstverdaus der zu trennenden Enzyme minimiert wird. Der Verzicht auf Gelfiltrationen ermöglicht femer die Durchführung der chromatographischen Stufen in einem weiten Temperaturbereich zwischen 4 und 25 °C, also auch bei Raumtemperatur. Femer umfasst das Verfahren auch keine affinitätschromatographischen Stufen, so dass die Gefahr des Ausblutens, also des Auswaschens der Affinitätsliganden des Chromatographiematerials nicht besteht. Schließlich sieht das erfindungsgemäße Verfahren auch keine Proteinfällungsschritte vor, die unerwünschte Strukturveränderungen der Proteine bewirken können. Hierdurch ergibt sich ferner der Vorteil, dass keine Detergenzien oder chaotrope Substanzen (Ammoniumsulfat, Polyethylenglycol, etc.) eingesetzt werden, deren nachträgliche Entfernung stets mit einem hohen Prozessaufwand verbunden ist.

Die verwendeten Chromatographiematerialien haben ferner den Vorteil, chemisch weitgehend inert zu sein und mit verhältnismäßig hoch konzentrierten Alkalilaugen, beispielsweise mit 3 molarer Natronlauge, gereinigt werden zu können. Dies gewährleistet eine sehr effektive Reinigung und somit einen Funktionserhalt der Materialien sowie eine gute Reproduzierbarkeit des Verfahrens. Die hohe Druckstabilität der Chromatographiematerialien gestattet zudem den Einsatz in Hochdruck-Flüssigchromatographie-Verfahren (HPLC). Aufgrund der Abwesenheit von toxikologisch bedenklichen, verfahrensbedingten Substanzen, wie zum Beispiel Affinitätsliganden oder Detergenzien, können die erfindungsgemäß aufgereinigten Proteine, insbesondere Typ I und/oder Typ II Kollagenase und/oder Clostripain und/oder neutrale Protease (Caseinase) besonders vorteilhaft für pharmazeutische oder biochemische Zwecke eingesetzt werden.

Weitere bevorzugte Ausgestaltungen der Erfindung ergeben sich aus den übrigen, in den Unteransprüchen genannten Merkmalen.

### Kurzbeschreibung der Zeichnung

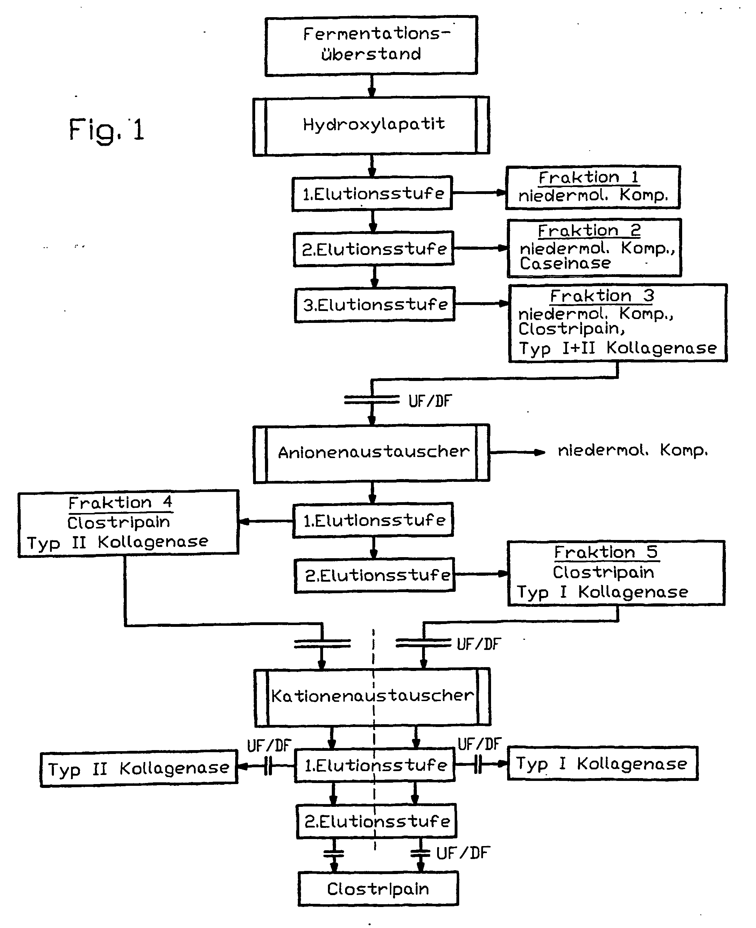

Im Folgenden wird die Erfindung mit Hilfe der Fig. 1 beispielhaft erläutert.

### Detaillierte Beschreibung der Erfindung und bester Weg zur Ausführung der Erfindung

Nach erfolgter Kultivierung von *Clostridium histolyticum* in einem Fermentationsmedium tierischen oder pflanzlichen Ursprungs werden die Zellen und sonstige nichtlösliche Bestandteile beispielsweise durch Zentrifugation oder Filtration vom Fermentationsüberstand abgetrennt. Der Fermentationsüberstand, der als Hauptbestandteile Typ I und Typ II Kollagenasen, Clostripain und Caseinase enthält, kann vor der chromatographischen Trennung dieser Proteine in üblicher Weise aufkonzentriert werden.

Der Fermentationsüberstand wird in einem ersten Schritt des Verfahrens auf eine mit keramischem Hydroxylapatitmaterial Typ I oder Typ II (CHT) gefüllte Chromatographiesäule gepumpt, wobei die genannten Enzyme neben verschiedenen anderen Komponenten an dem Hydroxylapatit binden. Bei Temperaturen von 4 bis 25 °C und einem pH-Wert von 6 bis 9 wird mittels Stufenelution bei linearen Flussraten > 300 cm/h eluiert. Dabei eignet sich ein Phosphatpuffer, der 0 bis 1000 mM eines Alkalihalogenides enthält, wobei die Phosphatkonzentration stufenweise von 10 bis 350 mM Phosphat erhöht wird. Alternativ oder zusätzlich kann der pH-Wert des Puffers schrittweise von 6 bis 9 erhöht werden. Es werden vorzugsweise drei Elutionsstufen durchgeführt. Die in der ersten Elutionsstufe erhaltene Fraktion 1 enthält ausschließlich niedermolekulare Bestandteile. Fraktion 2 der zweiten Elutionsstufe enthält neben niedermolekularen Komponenten neutrale Protease (Caseinase). Fraktion 3 der dritten Elutionsstufe enthält ebenfalls niedermolekulare Komponenten, das gesamte Clostripain sowie sämtliche Typ I und Typ II Kollagenasen.

Fraktion 3 wird beispielsweise mittels Ultrafiltration/Diafiltration oder Nanofiltration oder Dialyse entsalzt und gegebenenfalls aufkonzentriert. Anschließend wird die entsalzte Lösung in einem zweiten chromatographischen Schritt einer Anionenaustauschchromatographie unterzogen. Hierfür wird ein Chromatographiematerial auf Styrol/Divinylbenzolbasis verwendet, das beispielsweise mit einer quartären Ammoniumgruppe funktionalisiert ist. Dabei kann auf kommerziell erhältliche Materialien zurückgegriffen werden (zum Beispiel Source der Firma Pharmacia, POROS der Firma PerSeptive, Makroprep der Firma Biorad). Bereits bei der Beladung des Anionenaustauschers mit Fraktion 3 erfolgt eine Abtrennung kationischer oder nichtionischer, niedermolekularer Bestandteile. Die Elution der gebundenen Bestandteile erfolgt bei 4 bis 25 °C in einem gepufferten System im pH-Bereich von 7 bis 9,5 mittels Stufenelution bei einer linearen Flussrate oberhalb von 500 cm/h, wobei eine Alkali- oder Erdalkalihalogenidkonzentration von 1 bis 1000 mM und/oder der pH-Wert von 9,5 bis 6 schrittweise variiert wird. Vorzugsweise werden zwei Fraktionen getrennt, wobei Fraktion 4 der ersten Elutionsstufe Clostripain und Typ II Kollagenase und Fraktion 5 der zweiten Elutionsstufe ebenfalls Clostripain sowie Typ I Kollagenase enthält.

Fraktion 4 und Fraktion 5 werden getrennt voneinander in einer dritten chromatographischen Stufe einer Kationenaustauschchromatographie unterzogen. Dabei wird ebenfalls eine auf Styrol/Divinylbenzolmaterial basierende Säulenfüllung verwendet, welche hier jedoch mit einer Kationen bindenden Gruppe, beispielsweise SO₃H, funktionalisiert ist. Auch hier können die vorstehend genannten, kommerziellen Materialien verwendet werden. Die Elution des Kationenaustauschers wird bei 4 bis 25 °C in einem gepufferten System im pH-Bereich von 5,7 bis 7 bei linearen Flussraten von mindestens 500 cm/h mittels Stufenelution durchgeführt. Dabei wird im Elutionspuffer eine Alkali- beziehungsweise Erdalkalihalogenidkonzentration zwischen 0 und 300 mM und/oder der pH-Wert zwischen 5 und 7 schrittweise eingestellt. Die Elution wird vorzugsweise in zwei Stufen durchgeführt, wobei in der ersten Elutionsstufe die jeweilige Kollagenase und in der zweiten Elutionsstufe Clostripain erhalten wird.

### Ausführungsbeispiel

Eine Kultur von *Clostridium histolyticum* wird unter Verwendung eines tierischen oder pflanzlichen Nährmediums in Flüssigkultur nach Standardmethoden bis zu einer gewünschten Zelldichte fermentiert. Nach Abtrennung der Zellen mit üblichen Methoden, beispielsweise durch Zentrifugation oder Filtration, werden 2000 ml des konzentrierten Fermentationsüberstandes mit einer linearen Flussrate von 300 cm/h auf eine mit 1700 ml keramischem Hydroxylapatit Typ I gefüllte Chromatographiesäule gepumpt. Die an die Hydoxylapatitsäule gebundenen Komponenten werden bei 20 bis 25 °C mit einer linearen Flussrate von 300 cm/h in drei Stufen mit Phosphatpuffer eluiert, wobei die Phosphatkonzentration schrittweise erhöht wird. In der ersten Elutionsstufe wird mit zirka 10 CV (Säulenvolumina) mit 10 mM Phosphatpuffer/100 mM NaCl eluiert, wobei die hauptsächlich niedermolekulare Bestandteile enthaltende Fraktion 1 erhalten wird. Anschließend wird mit etwa 3 CV 60 mM Phosphatpuffer/100 mM NaCl eluiert und Fraktion 2 erhalten, die Caseinase und ebenfalls niedermolekulare Komponenten enthält. Für die dritte Elutionsstufe wird mit zirka 5 CV 200 mM Phosphatpuffer/100 mM NaCl eluiert. Die hierbei gesammelte Fraktion 3 enthält die Enzyme Clostripain und sämtliche Typ I und Typ II Kollagenasen. Fraktion 2 wird entsalzt und lyophilisiert. Eine Abtrennung der niedermolekularen Bestandteile von Caseinase kann gegebenenfalls mit Standardmethoden erreicht werden.

Fraktion 3 wird mittels Ultrafiltration/Diafiltration oder Nanofiltration oder Dialyse entsalzt und gegebenenfalls aufkonzentriert. Anschließend wird Fraktion 3 mit einem geeigneten Puffer, beispielsweise 500 mM Tris pH 9,0 - 9,3, auf pH 9,0 - 9,3 eingestellt. Die gepufferte Lösung wird bei 20 - 25 °C mit einer linearen Flussrate von zirka 1000 cm/h über eine als Anionenaustauscher funktionalisierte Styrol/Divinylbenzolsäule (POROS 50 PI der Firma PerSeptive) gepumpt. Anschließend wird die Säule mit zirka 5 CV Tris-Puffer gewaschen. Die Elution erfolgt bei 20 - 25 °C und einer linearen Flussrate von zirka 1000 cm/h in zwei Elutionsstufen mit steigender Salzkonzentration. Fraktion 4 wird durch Elution mit 40 mM Tris Puffer/6 mM CaCl₂/30 mM NaCl pH 9,0 - 9,3 erhalten und enthält die Enzyme Clostripain und Typ II Kollagenase. Fraktion 5, die ebenfalls Clostripain sowie Typ I Kollagenase enthält, wird in der zweiten .Elutionsstufe durch Elution mit 40 mM Tris/6 mM CaCl₂/70 mM NaCl pH 9,0 - 9,3 erhalten.

Fraktion 4 und Fraktion 5 werden beispielsweise durch Dialyse für 24 Stunden gegen 50 l H₂O entsalzt und mit 50 mM MES-Puffer auf pH 5,9 - 6,1 eingestellt. Beide Fraktionen werden getrennt voneinander an einer Kationenaustauschersäule auf Styrol/Divinylbenzolmaterial (zum Beispiel POROS 50 HS der Firma PerSeptive) chromatographiert. Dafür werden die Fraktionen mit einer linearen Flussrate von zirka 700 cm/h bei 20 - 25 °C auf die Säule geladen und unter gleichen Bedingungen eluiert. Die Elution von Typ II Kollagenase aus Fraktion 4 bzw. Typ I Kollagenase aus Fraktion 5 erfolgt jeweils in einer ersten Elutionsstufe mit 10 mM MES-Puffer/20 - 40 mM NaCl pH 5,9 - 6,1 bei 20 - 25 °C. Clostripain aus beiden Fraktionen wird jeweils anschließend mit 10 mM MES-Puffer/90 - 110 mM NaCl pH 5,9 - 6,1 eluiert. Die Clostripain enthaltenden Lösungen werden vereinigt. Alle Lösungen werden entsalzt, gegen 2 mM CaAc₂ dialysiert und anschließend lyophilisiert.

Eine Bestimmung der jeweiligen Enzymaktivitäten der so erhaltenen Enzyme erfolgte nach bekannten Methoden (s. Tabelle). Ferner wurde die Reinheit der Enzyme Typ I Kollagenase, Typ II Kollagenase und Clostripain mittels reversed phase Chromatographie bestimmt. Die Ergebnisse sind nachfolgend in der Tabelle zusammengefasst:

**Tabelle:**

| | Aktivität | Reinheit^{e} |
|---|---|---|
| Typ II Kollagenase | 18100 U/g ^{a} | ca. 82 % |
| Typ I Kollagenase | 5180 U/mg ^{b} | ca. 85 % |
| Clostripain | 322 U/mg ^{c} | ca. 90 % |
| Neutrale Protease | 1560 U/mg ^{d} | |

| | | |
|---|---|---|
| ^{a} bestimmt nach Wünsch E., Heidrich H.-G.; Z. Physiol. Chem. 333, 149 - 151, 1963; | | |
| ^{b} bestimmt nach Doi, Shibata, Matoba; Anal. Biochem. 118, 173 - 184, 1981; | | |
| ^{c} bestimmt nach Mitchel, Harrington; Methods Enzymol., 19, 635 - 642, 1970; | | |
| ^{d} bestimmt nach Moore, Stein; Biol. Chem. 176, 367, 1948; | | |
| ^{e} bestimmt mit RPC. | | |

## Patentansprüche

1. Verfahren zur teilweisen oder vollständigen Aufreinigung mindestens eines in einem Fermentationsüberstand von *Clostridium histolyticum* enthaltenen Enzyms,
**dadurch gekennzeichnet,**
**dass** die Enzyme des Fermentationsüberstandes durch ein Chromatographieverfahren unter ausschließlicher Verwendung erster Chromatographiematerialien auf Basis von keramischem, Hydroxylapatit und zweiter Chromatographiematerialien auf Styrol/Divinylbenzolbasis aufgetrennt werden, wobei
a.) das Chromatographieverfahren eine erste chromatographische Stufe an gesintertem Hydroxylapatitmaterial, eine zweite chromatographische Stufe an einem Anionenaustauschermaterial auf Styrol/Divinylbenzolbasis und gegebenenfalls eine dritte chromatographische Stufe an einem Kationenaustauschermaterial auf Styrol/Divinylbenzolbasis umfasst, wobei die Elution an dem gesinterten Hydroxylapatitmaterial bei Flussraten von mindestens 200 cm/h und insbesondere von mindestens 300 cm/h, die Elution an dem Styrol/Divinylbenzolmaterial bei Flussraten von mindestens 500 cm/h, insbesondere von mindestens 1000 cm/h, durchgeführt wird,
b.) die erste chromatographische Stufe in mindestens zwei Elutionsstufen durchgeführt wird, wobei neutrale Protease (Caseinase) von Typ 1 und Typ II Kollagenase und Clostripain abgetrennt wird,
c.) eine im wesentlichen von Caseinase befreite Fraktion aus der ersten chromatographischen Stufe der zweiten chromatographischen Stufe mit mindestens zwei Elutionsstufen unterzogen wird, wobei Typ I Kollagenase und Typ II Kollagenase voneinander getrennt werden,
d.) eine Typ I Kollagenase enthaltene Fraktion aus der zweiten chromatographischen Stufe der dritten chromatographischen Stufe mit mindestens zwei Elutionsstufen unterworfen wird, wobei Typ 1 Kollagenase von Clostripain abgetrennt wird,
e.) eine Typ II Kollagenase enthaltene Fraktion aus der zweiten chromatographischen Stufe der dritten chromatograhischen Stufe mit mindestens zwei Elutionsstufen unterworfen wird, wobei Typ II Kollagenase von Clostripain abgetrennt wird,
f.) die chromatographischen Stufen bei Temperaturen zwischen 4° und 25° C durchgeführt werden.

2. Verfahren nach Anspruch 1,
**dadurch gekennzeichnet,**
**dass** das Enzym Typ I Kollagenase und/oder Typ II Kollagenase und/oder Clostripain und/oder neutrale Protease (Caseinase) ist.

3. Verfahren nach einem der vorhergehenden Ansprüche,
**dadurch gekennzeichnet,**
**dass** gesintertes Hydroxylapatit verwendet wird.

4. Verfahren nach einem der vorhergehenden Ansprüche,
**dadurch gekennzeichnet,**
**dass** das Chromatographieverfahren ausschließlich Schritte umfasst, die auf elektrostatischen Wechselwirkungen und/oder auf lonenbindung und/oder ionischer Komplexbindung beruhen.

5. Verfahren nach Anspruch 4,
**dadurch gekennzeichnet,**
**dass** das Chromatographieverfahren mindestens eine anionenaustauschchromatographische Stufe und/oder mindestens eine kationenaustauschchromatographische Stufe und/oder mindestens eine hydroxylapatitchromtographische Stufe umfasst.

6. Verfahren nach einem der vorhergehenden Ansprüche,
**dadurch gekennzeichnet,**
**dass** mindestens eine Chromatographiestufe, insbesondere alle Chromatographiestufen, in Form einer Stufenelution durchgeführt wird.

7. Verfahren nach einem der vorhergehenden Ansprüche,
**dadurch gekennzeichnet,**
**dass** das Chromatographieverfahren zwei- oder dreistufig ist.

8. Verfahren nach Anspruch 7,
**dadurch gekennzeichnet,**
**dass** das Chromatographieverfahren eine erste chromatographische Stufe an gesintertem Hydroxylapatitmaterial, eine zweite chromatographische Stufe an einem Anionenaustauschermaterial auf Styrol/Divinylbenzolbasis und ggf. eine dritte chromatographische Stufe an einem Kationenaustauschermaterial auf Styrol/Divinylbenzolbasis umfasst.

9. Verfahren nach einem der Ansprüche 7 oder 8,
**dadurch gekennzeichnet,**
**dass** die erste chromatographische Stufe in mindestens zwei Elutionsstufen durchgeführt wird, wobei neutrale Protease (Caseinase) von Typ I und Typ II Kollagenase und Clostripain abgetrennt wird.

10. Verfahren nach einem der Ansprüche 7 bis 9,
**dadurch gekennzeichnet,**
**dass** eine im Wesentlichen von Caseinase befreite Fraktion aus der ersten chromatographischen Stufe der zweiten chromatographischen Stufe mit mindestens zwei Elutionsstufen unterzogen wird, wobei Typ I Kollagenase und Typ II Kollagenase voneinander getrennt werden.

11. Verfahren nach einem der Ansprüche 7 bis 10,
**dadurch gekennzeichnet,**
**dass** eine Typ I Kollagenase enthaltende Fraktion aus der zweiten chromatographischen Stufe der dritten chromatographischen Stufe mit mindestens zwei Elutionsstufen unterworfen wird, wobei Typ I Kollagenase von Clostripain abgetrennt wird.

12. Verfahren nach einem der Ansprüche 8 bis 11,
**dadurch gekennzeichnet,**
**dass** eine Typ II Kollagenase enthaltende Fraktion aus der zweiten chromatographischen Stufe der dritten chromatographischen Stufe mit mindestens zwei Elutionsstufen unterworfen wird, wobei Typ II Kollagenase von Clostripain abgetrennt wird.

13. Verfahren nach einem der vorhergehenden Ansprüche,
**dadurch gekennzeichnet,**
**dass** die Elution bei linearen Flussraten durchgeführt wird.

14. Verfahren nach einem der vorhergehenden Ansprüche,
**dadurch gekennzeichnet,**
**dass** die Elution an dem gesinterten Hydroxylapatitmaterial bei Flussraten von mindestens 200 cm/h, insbesondere von mindestens 300 cm/h, durchgeführt wird.

15. Verfahren nach einem der vorhergehenden Ansprüche,
**dadurch gekennzeichnet,**
**dass** die Elution an dem Styrol/Divinylbenzolmaterial bei Flussraten von mindestens 500 cm/h, insbesondere von mindestens 1000 cm/h, durchgeführt wird.

16. Verfahren nach einem der vorhergehenden Ansprüche,
**dadurch gekennzeichnet,**
**dass** das Verfahren keine Gelfiltrationsschritte umfasst.

17. Verfahren nach einem der vorhergehenden Ansprüche,
**dadurch gekennzeichnet,**
**dass** das Verfahren keine Proteinfällungsschritte umfasst.

18. Verfahren nach einem der vorhergehenden Ansprüche,
**dadurch gekennzeichnet,**
**dass** das Verfahren abreichemde Schritte für niedermolekulare Substanzen, Wirtszellproteine, Endotoxine und/oder Nukleinsäuren, insbesondere DNA, umfasst.

19. Verfahren nach einem der vorhergehenden Ansprüche,
**dadurch gekennzeichnet,**
**dass** die chromatographischen Stufen bei Temperaturen zwischen 4 und 25 °C durchgeführt werden.

20. Verfahren nach einem der vorhergehenden Ansprüche,
**dadurch gekennzeichnet,**
**dass** zertifizierte Chromatographiemedien verwendet werden.

21. Verfahren nach einem der vorhergehenden Ansprüche,
**dadurch gekennzeichnet,**
**dass** druckstabile Chromatographiematerialien verwendet werden.

22. Verfahren nach einem der vorhergehenden Ansprüche,
**dadurch gekennzeichnet,**
**dass** eine Reinigung der Chromatographiematerialien mit einer konzentrierten Alkalilauge, insbesondere mit 3 molarer Natronlauge, durchgeführt wird.

23. Verfahren nach einem der vorhergehenden Ansprüche,
**dadurch gekennzeichnet,**
**dass** die Chromatographiestufen als Säulenchromatographie durchgeführt werden.

24. Verfahren nach einem der vorhergehenden Ansprüche,
**dadurch gekennzeichnet,**
**dass** Typ II Kollagenase mit einer spezifischen Aktivität von mindestens 13000 U/g, insbesondere mindestens 18000 U/g, erhalten wird.

25. Verfahren nach einem der vorhergehenden Ansprüche,
**dadurch gekennzeichnet,**
**dass** Typ I Kollagenase mit einer spezifischen Aktivität von mindestens 3000 U/mg, insbesondere mindestens 5000 U/mg, erhalten wird.

26. Verfahren nach einem der vorhergehenden Ansprüche,
**dadurch gekennzeichnet,**
**dass** Clostripain mit einer spezifischen Aktivität von mindestens 200 U/mg, insbesondere mindestens 300 U/mg, erhalten wird

27. Verfahren nach einem der vorhergehenden Ansprüche,
**dadurch gekennzeichnet,**
**dass** neutrale Protease (Caseinase) mit einer spezifischen Aktivität von mindestens 1200 U/mg, insbesondere mindestens 1500 U/mg, erhalten wird.

28. Verfahren nach einem der vorhergehenden Ansprüche,
**dadurch gekennzeichnet,**
**dass** Typ II Kollagenase und/oder Typ I Kollagenase und/oder Clostripain jeweils mit einer Reinheit von mindestens 70 %, ins besondere mindestens 80 %, erhalten werden.

## Claims

1. Method for the partial or complete purification of at least one enzyme contained in an excess fermentation of *Clostridium histolyticum*,
**characterized in**
**that** the enzymes of the excess fermentation are separated by a chromatographic method by exclusively using first chromatographic materials on the base of ceramic hydroxylapatite and second chromatographic materials on styrene/divinylbenzene base, whereby
a.) the chromatography method comprises a first chromatography stage on sintered hydroxylapatite material, a second chromatography stage on an anion exchange material on styrene/divinylbenzene base and eventually a third chromatography stage on a cation exchange material on styrene/divinylbenzene base, whereby the elution takes place on the sintered hydroxylapatite material at flow rates of at least 200 cm/h and in particular of at least 300 cm/h, the elution on the styrene/divinylbenzene material at flow rates of at least 500 cm/h, in particular of at least 1000 cm/h,
b.) the first chromatography stage is carried out in at least two elution stages, whereby neutral protease (caseinase) is separated from type I and type II collagenase and clostripain,
c.) a fraction from the first chromatography stage substantially freed from caseinase is submitted to the second chromatography stage with at least two elution stages, whereby type I collagenase and type II collagenase are separated from each other,
d.) a fraction from the second chromatography stage containing type I collagenase is submitted to the third chromatography stage with at least two elution stages, whereby type I collagenase is separated from clostripain,
e.) a fraction from the second chromatography stage containing type II collagenase is submitted to the third chromatography stage with at least two elution stages, whereby type II collagenase is separated from clostripain,
f.) the chromatography stages are carried out at temperatures between 4° and 25° C.

2. Method according to claim 1,
**characterized in**
**that** the enzyme is type I collagenase and/or type II collagenase and/or clostripain and/or neutral protease (caseinase).

3. Method according to any of the preceding claims,
**characterized in**
**that** sintered hydroxylapatite is used.

4. Method according to any of the preceding claims,
**characterized in**
**that** the chromatography method comprises exclusively steps which are based on electrostatic interactions and/or on ion bond and/or ionic complexing.

5. Method according to claim 4,
**characterized in**
**that** the chromatography method comprises at least one anion exchange chromatography stage and/or at least one cation exchange chromatography stage and/or at least one hydroxylapatite chromatography stage.

6. Method according to any of the preceding claims,
**characterized in**
**that** at least one chromatography stage, in particular all the chromatography stages, are carried out as a stepwise elution.

7. Method according to any of the preceding claims
**characterized in**
**that** the chromatography method has two or three stages.

8. Method according to claim 7,
**characterized in**
**that** the chromatography method comprises a first chromatography stage on sintered hydroxylapatite material, a second chromatography stage on an anion exchange material on styrene/divinylbenzene base and eventually a third chromatography stage on a cation exchange material on styrene/divinylbenzene base.

9. Method according to any of the claims 7 or 8,
**characterized in**
**that** the first chromatography stage is carried out in at least two elution stages, whereby neutral protease (caseinase) is separated from type I and type II collagenase and clostripain.

10. Method according to any of the claims 7 to 9,
**characterized in**
**that** one fraction from the first chromatography stage substantially freed from caseinase is submitted to the second chromatography stage with at least two elution stages, whereby type I collagenase and type II collagenase are separated from each other.

11. Method according to any of the claims 7 to 10,
**characterized in**
**that** one fraction from the second chromatography stage containing type I collagenase is submitted to the third chromatography stage with at least two elution stages, whereby type I collagenase is separated from clostripain.

12. Method according to any of the claims 8 to 11,
**characterized in**
**that** one fraction from the second chromatography stage containing type II collagenase is submitted to the third chromatography stage with at least two elution stages, whereby type II collagenase is separated from clostripain.

13. Method according to any of the preceding claims,
**characterized in**
**that** the elution is carried out at linear flow rates.

14. Method according to any of the preceding claims,
**characterized in**
**that** the elution on the sintered hydroxylapatite material is carried out at flow rates of at least 200 cm/h, in particular of at least 300 cm/h.

15. Method according to any of the preceding claims,
**characterized in**
**that** the elution on styrene/divinylbenzene material is carried out at flow rates of at least 500 cm/h, in particular of at least 1000 cm/h.

16. Method according to any of the preceding claims,
**characterized in**
**that** the method does not comprise any gel filtration steps.

17. Method according to any of the preceding claims,
**characterized in**
**that** the method does not comprise any protein precipitation steps.

18. Method according to any of the preceding claims,
**characterized in**
**that** the method comprises degrading steps for low molecular substances, host cell proteines, endotoxins and/or nucleic acids, in particular DNA.

19. Method according to any of the preceding claims,
**characterized in**
**that** the chromatography stages are carried out at temperatures between 4 and 25° C.

20. Method according to any of the preceding claims,
**characterized in**
**that** certified chromatographic media are used.

21. Method according to any of the preceding claims,
**characterized in**
**that** pressure stable chromatographic materials are used.

22. Method according to any of the preceding claims,
**characterized in**
**that** a purification of the chromatographic materials is carried out with a concentrated alkalyne lie, in particular with 3-molar caustic soda lye.

23. Method according to any of the preceding claims,
**characterized in**
**that** the chromatography stages are carried out as column chromatography.

24. Method according to any of the preceding claims,
**characterized in**
**that** type II collagenase with a specific activity of at least 13000 U/g, in particular of at least 18000 U/g is obtained.

25. Method according to any of the preceding claims,
**characterized in**
**that** type I collagenase with a specific activity of at least 3000 U/g, in particular of at least 5000 U/g is obtained.

26. Method according to any of the preceding claims,
**characterized in**
**that** clostripain with a specific activity of at least 200 U/g, in particular of at least 300 U/g is obtained.

27. Method according to any of the preceding claims,
**characterized in**
**that** neutral protease (caseinase) with a specific activity of at least 1200 U/g, in particular of at least 1500 U/g is obtained.

28. Method according to any of the preceding claims,
**characterized in**
**that** type II collagenase and/or type I collagenase and/or clostripain are obtained respectively with a purity of at least 70 %, in particular of at least 80 %.

## Revendications

1. Procédé de purification partielle ou complète d'au moins une enzyme contenue dans un excès de fermentation de *Clostridium histolyticum*,
**caractérisé en ce**
**que** les enzymes de l'excès de fermentation sont séparés par un procédé de chromatographie en utilisant exclusivement des premiers matériaux de chromatographie à base d'hydroxylapatite céramique et de seconds matériaux de chromatographie à base de styrène/divinylbenzène,
a.) le procédé de chromatographie comprenant une première phase chromatographique sur du matériau à base d'hydroxylapatite fritté, une seconde phase chromatographique sur un matériau échangeur d'anions à base de styrène/divinylbenzène et, éventuellement, une troisième phase chromatographique sur un matériau échangeur de cations à base de styrène/divinylbenzène, l'élution étant effectuée sur le matériau à base d'hydroxylapatite fritté à des vitesses d'écoulement d'au moins 200 cm/h et en particulier d'au moins 300 cm/h, l'élution sur du matériau à base de styrène/divinylbenzène à des vitesses d'écoulement d'au moins 500 cm/h, en particulier d'au moins 1000 cm/h,
b.) la première phase chromatographique est effectuée dans au moins deux phases d'élution, la protéase neutre (caséisase) étant séparée de la collagénase de type I et de type II et de la clostripaïne,
c.) une fraction substantiellement exempte de caséinase de la première phase chromatographique étant soumise à la seconde phase chromatographique avec au moins deux phases d'élution, la collagénase de type I et la collagénase de type II étant séparées l'une de l'autre,
d.) une fraction contenant de la collagénase de type I de la seconde phase chromatographique étant soumise à la troisième phase chromatographique avec au moins deux phases d'élution, la collagénase de type I étant séparée de la clostripaïne,
e.) une fraction contenant de la collagnénase de type II de la seconde phase chromatographique étant soumise à la troisième phase chromatographique avec au moins deux phases d'élution, la collagénase de type II étant séparée de la clostripaïne,
f.) les phases chromatographiques étant effectuées à des températures entre 4° et 25° C.

2. Procédé selon la revendication 1,
**caractérisé en ce**
**que** l'enzyme est de la collagénase de type I et/ou de la collagénase de type II et/ou de la clostripaïne et/ou une protéase neutre (caséinase).

3. Procédé selon l'une des revendications précédentes,
**caractérisé en ce**
**que** l'on utilise de l'hydroxylapatite fritté.

4. Procédé selon l'une des revendications précédentes,
**caractérisé en ce**
**que** le procédé de chromatographie comprend exclusivement des étapes qui reposent sur des interactions électrostatiques et/ou sur la liaison ionique et/ou la liaison de complexe ionique.

5. Procédé selon la revendication 4.
**caractérisé en ce**
**que** le procédé chromatographique comprend au moins une étape chromatographique d'échange d'anions et/ou au moins une étape chromatographique d'échange de cations et/ou au moins une étape chromatographique avec de l'hydroxylapatite.

6. Procédé selon l'une des revendications précédentes,
**caractérisé en ce**
**qu'**au moins une étape chromatographique, qu'en particulier toutes les étapes chromatographiques, sont effectuées sous forme d'une élution en étapes.

7. Procédé selon l'une des revendications précédentes,
**caractérisé en ce**
**que** le procédé de chromatographie est à deux ou trois phases.

8. Procédé selon la revendication 7,
**caractérisé en ce**
**que** le procédé de chromatographie comprend une première phase chromatographique sur du matériau à base d'hydroxylapatite fritté, une seconde phase chromatographique sur un matériau échangeur d'anions à base de styrène/divinylbenzène et, éventuellement, une troisième phase chromatographique sur un matériau échangeur de cations à base de styrène/divinylbenzène.

9. Procédé selon l'une des revendications 7 ou 8,
**caractérisé en ce**
**que** la première phase chromatographique est effectuée dans au moins deux phases d'élution, la protéase neutre (caséinase) étant séparée de la collagénase de type I et de type II et de la clostripaïne.

10. Procédé selon l'une des revendications 7 à 9,
**caractérisé en ce**
**qu'**une fraction de la première phase chromatographique substantiellement exempte de caséinase est soumise à la seconde phase chromatographique avec au moins deux phases d'élution, la collagénase de type I et la collagénase de type II étant séparées l'une de l'autre.

11. Procédé selon l'une des revendications 7 à 10,
**caractérisé en ce**
**qu'**une fraction qui contient de la collagénase de type I de la seconde phase chromatographique est soumise à la troisième phase chromatographique avec au moins deux phases d'élution, la collagénase de type I étant séparée de la clostripaïne.

12. Procédé selon l'une des revendications 8 à 11,
**caractérisé en ce**
**qu'**une fraction qui contient de la collagénase de type II de la seconde phase chromatographique est soumise à la troisième phase chromatographique avec au moins deux phases d'élution, la collagénase de type II étant séparée de la clostripaïne.

13. Procédé selon l'une des revendications précédentes,
**caractérisé en ce**
**que** l'élution est effectuée pour des vitesses d'écoulement linéaires.

14. Procédé selon l'une des revendications précédentes,
**caractérisé en ce**
**que** l'élution sur le matériau à base d'hydroxylapatite fritté est effectuée à des vitesses d'écoulement d'au moins 200 cm/h, en particulier d'au moins 300 cm/h.

15. Procédé selon l'une des revendications précédentes,
**caractérisé en ce**
**que** l'élution sur le matériau à base de styrène/divinylbenzène est effectuée à des vitesses d'écoulement d'au moins 500 cm/h, en particulier d'au moins 1000 cm/h.

16. Procédé selon l'une des revendications précédentes,
**caractérisé en ce**
**que** le procédé ne comprend pas d'étapes de filtration de gel.

17. Procédé selon l'une des revendications précédentes,
**caractérisé en ce**
**que** le procédé ne comprend pas d'étapes de précipitation de protéines.

18. Procédé selon l'une des revendications précédentes,
**caractérisé en ce**
**que** le procédé comprend des étapes d'appauvrissement pour des substances de faible poids moléculaire, des protéines de cellules-hôtes, des endotoxines et/ou des acides nucléiques, en particulier de l'ADN.

19. Procédé selon l'une des revendications précédentes,
**caractérisé en ce**
**que** les phases chromatographiques sont effectuées à des températures entre 4 et 25° C.

20. Procédé selon l'une des revendications précédentes,
**caractérisé en ce**
**que** des milieux de chromatographie certifiés sont utilisés.

21. Procédé selon l'une des revendications précédentes,
**caractérisé en ce**
**que** des matériaux de chromatographie stables à la pression sont utilisés.

22. Procédé selon l'une des revendications précédentes,
**caractérisé en ce**
**qu'**un nettoyage des matériaux de chromatographie est effectué avec une lessive alcaline concentrée, en particulier avec de la lessive de soude de poids molaire 3.

23. Procédé selon l'une des revendications précédentes,
**caractérisé en ce**
**que** les phases de chromatographie sont effectuées comme chromatographie sur colonne échangeuse.

24. Procédé selon l'une des revendications précédentes,
**caractérisé en ce**
**que** l'on obtient de la collagénase de type II avec une activité spécifique d'au moins 13000 U/g, en particulier d'au moins 18000 U/g.

25. Procédé selon l'une des revendications précédentes,
**caractérisé en ce**
**que** l'on obtient de la collagénase de type I avec une activité spécifique d'au moins 3000 U/g, en particulier d'au moins 5000 U/g.

26. Procédé selon l'une des revendications précédentes,
**caractérisé en ce**
**que** l'on obtient de la clostripaïne avec une activité spécifique d'au moins 200 U/mg, en particulier d'au moins 300 U/mg.

27. Procédé selon l'une des revendications précédentes,
**caractérisé en ce**
**que** l'on obtient de la protéase neutre (caséinase) avec une activité spécifique d'au moins 1200 U/g, en particulier d'au moins 1500 U/mg.

28. Procédé selon l'une des revendications précédentes,
**caractérisé en ce**
**que** l'on obtient de la collagénase de type II et/ou de la collagénase de type I et/ou de la clostripaïne avec une pureté d'au moins 70 %, en particulier d'au moins 80 %.
